**Europäisches Patentamt**

(19) **European Patent Office**

**Office européen des brevets**

(11) Publication number : **0 408 063 B1**

# (12) EUROPEAN PATENT SPECIFICATION

(45) Date of publication of patent specification :
27.10.93 Bulletin 93/43

(51) Int. Cl.$^5$ : **A23J 3/34**

(21) Application number : **90113464.3**

(22) Date of filing : **13.07.90**

(54) A process for the production of hydrolyzed vegetable proteins and the product therefrom.

(30) Priority : **14.07.89 US 380182**

(43) Date of publication of application :
**16.01.91 Bulletin 91/03**

(45) Publication of the grant of the patent :
**27.10.93 Bulletin 93/43**

(84) Designated Contracting States :
**AT BE CH DE DK ES FR GB GR IT LI NL SE**

(56) References cited :
**EP-A- 0 226 769**
**EP-A- 0 361 595**
**US-A- 4 757 007**
**JAPS; & JP-A-63 074 465 (DAINIPPON SEITO K.K.) 04-04-1988**
**Complete English translation of JP-A-63-74465**

(73) Proprietor : **CPC INTERNATIONAL INC.**
**International Plaza P.O. Box 8000**
**Englewood Cliffs New Jersey 07632 (US)**

(72) Inventor : **Hamm, Donald J.**
**57 Commonwealth Avenue**
**New Providence, NJ 07974 (US)**

(74) Representative : **Lederer, Franz, Dr. et al**
**Lederer, Keller & Riederer, Patentanwälte,**
**Lucile-Grahn-Strasse 22**
**D-81675 München (DE)**

EP 0 408 063 B1

## Description

BACKGROUND OF THE INVENTION

FIELD OF THE INVENTION

This invention relates to a process for the production of hydrolyzed vegetable proteins containing no detectable levels of monochloropropanol. The resultant hydrolyzed vegetable protein is clean and bland in flavor and exhibits substantial flavor enhancement characteristics.

DESCRIPTION OF THE PRIOR ART

The preparation of conventional hydrolyzed vegetable proteins (HVPs) is generally carried out by acid hydrolysis with hydrochloric acid under refluxing conditions, specifically using 6M hydrochloric acid at 109°C and atmospheric pressure. It has been demonstrated that hydrolyzing vegetable proteins at these conditions results in the chlorination of glycerol, which is derived from the residual fatty substances present in the crude protein, to produce monochloropropanols (MCPs) and dichloropropanols (DCPs). As MCP and DCP exhibit questionable properties and characteristics, their presence is not desired in food products. DCP is readily removed during the evaporation or concentration steps of standard processes. Unfortunately, MCP is not removed, but is concentrated in the finished product, and therefore, additional processing steps must be taken to remove the MCP from the finished product.

In a conventional acid hydrolysis process for preparing HVPs, the formation of MCP and DCP can be avoided by using sulfuric or phosphoric acid in place of hydrochloric acid. However, the HVPs produced by hydrolyzing with sulfuric or phosphoric acid are of an inferior quality in that they exhibit a bitter flavor.

The specific problem is that MCP is derived during conventional acid hydrolysis from the chlorination of the glycerol derived from the residual fatty substances which are present in crude proteins. As an example, vital wheat gluten which is approximately 75% protein, also contains 5.0 to 9.5% fat and other lipid materials, is an abundant source of glycerol in the form of a complex mixture of mono-, di- and tri-glycerides, phospholipids and glycolipids. Numerous factors which are believed to effect the formation of MCP include the presence of high concentrations of chloride ions, high amounts of excess acid, high refluxing temperatures and long reaction times. It is thought that the bound glycerol is more active in forming MCPs than unbound glycerol.

Much is also known about the use of enzymes to hydrolyze vegetable proteins for food use, but not for the purpose of flavor enhancement. What is taught in the existing art is generally directed toward producing functionally improved proteins, such as eliminating bitter peptide formation during enzyme hydrolysis as shown in U.S. Patent No. 4,636,388. Specifically, the patent discloses a low ash protein product which is particularly adapted for enzymatic hydrolysis. A dispersion of protein is gelled and then washed in particulate form in a liquid in order to allow a portion of the non-proteinaceous material to defuse from the gel into the liquid, and then the liquid is separated from the gel. The pretreated product is then hydrolyzed enzymatically, preferably with fungal protease and pancreatin.

U.S. Patent No. 4,757,007, discloses and claims a process for the preparation of hydrolyzed products of soy protein by partially hydrolyzing soy protein with protease, and then separating the resulting hydrolyzed products by using a 5% aqueous solution of trichloroacetic acid. The portion of hydrolyzed protein with low solubility possesses excellent emulsifying properties, while the one with high solubility possesses excellent foaming properties.

In U.S. Patent No. 3,830,942, a soluble protein product is produced which is particularly useful in highly acidic foods, and an insoluble protein product is prepared which is used in preparing protein enriched bakery goods. The patent discloses the method for producing the two products by forming an aqueous solution of defatted oleaginous seed materials, adjusting the pH of the slurry to the isoelectric point of the seed materials, heating the slurry to elevated temperatures, adding an enzyme to the slurry, agitating the mixture during hydrolysis of the material and thereafter, separating the hydrolyzed and unhydrolyzed portions of the protein product.

Although enzyme hydrolysis and acid hydrolysis are generally separate procedures, one patent has been found which discloses the combination of acid and enzyme hydrolysis to obtain a protein hydrolyzate. In USSR Patent Application No. 442800, a method of obtaining a preparation for parenteral protein feeding is taught. A method is disclosed wherein the raw protein material undergoes enzymatic cleavage, followed by acid hydrolysis with 5.0% sulfuric acid (4.0N), in a carbon dioxide atmosphere. Thereafter, the hydrolyzate is passed through an anion exchange column, treated with aluminum hydroxide and passed through a column containing cation exchange resin. The acid hydrolysis takes place at about 100°C for about seven (7) hours.

In JP 63-74 465 (1988) a seasoning production method is disclosed wherein gluten meal is treated with an enzyme and the water-soluble extract obtained therefrom is hydrolysed with acid for about 24 hours.

Many attempts have been made over the years to produce hydrolyzed vegetable protein products which are used for various purposes, however, to date no process has been taught which produces a hydrolyzed vegetable protein with reduced or non-existent levels of MCP or DCP due to preventing MCP and DCP production by controlling the parameters of the acid hydrolysis, and which exhibits substantial flavor enhancement characteristics.

## SUMMARY OF THE INVENTION

The present invention relates to the production of hydrolyzed vegetable proteins which contain no detectable levels of MCP. This result is achieved by a unique process which combines two methods of hydrolysis of the vegetable protein, enzymatic hydrolysis and mild acid hydrolysis. The hydrolyzates which result from this process are clean and bland in flavor, exhibit substantial flavor enhancement characters, and contain substantial amounts of monosodium glutamate, up to 25% by weight, on a dry weight basis.

The process for producing hydrolyzed vegetable proteins with no detectable level of MCP begins with the hydrolyzing of the protein by adding it to an aqueous solution of at least one protease. The resulting hydrolyzed soluble protein is then separated from the insoluble mass. Thereafter, acid is added and the mixture is heated, providing an acidified hydrolyzate, which is then neutralized.

It is believed that the enzyme hydrolysis step contributes to reducing the MCP and DCP formation by solubilizing the protein away from the majority of the non-proteinaceous components of the crude protein. This is believed to result in a significant reduction in the level of available glycerol containing fatty substances and thereby reduces the level of key substrates required for MCP formation during the subsequent acid hydrolysis step. Another function of the enzyme hydrolysis is to act on the protein to release small peptides and amino acids.

The acid hydrolysis step also contributes to the decrease in MCP level, as it is mild acid hydrolysis which is used. The conditions at which the acid hydrolysis, or deamidation take place, are significantly milder than those used in conventional processes. Specifically, the mild acid hydrolysis is carried out at significantly lower acid concentrations, at lower temperatures and for shorter periods of time than the conventional hydrolysis process. By controlling the conditions, deamidation preferentially occurs; ie, the amide linkages are hydrolyzed, but the peptide bond hydrolysis is controlled or minimized. It is believed that these conditions, combined with the reduced fat levels from the enzyme hydrolysis, are responsible for the lack of formation of MCPs in the finished product.

## DETAILED INVENTION

The present process comprises a number of steps for the hydrolyzing of a vegetable protein to result in a product which contains no detectable level of MCP. The term "no detectable level" as used herein means that there is no detectable level as measured by gas chromotagraphy (GC) with a sensitivity to levels as low as 2 ppm.

Specifically, a vegetable protein is hydrolyzed by adding it to an aqueous solution of at least one protease. The vegetable protein can be any one of the available vegetable proteins, such as, but not limited to, oil seed proteins (soy, peanut, sunflower, cotton seed), plasma proteins or leaf proteins. The preferred protein for producing savory flavors with substantial flavor enhancing properties is wheat gluten, due to its high glutamic acid content, present mostly as glutamine.

The protein is added to an aqueous solution of at least one endoprotease, which can be acidic, neutral or alkaline in form. The protease is chosen dependent upon a number of parameters for the particular enzyme/substrate combination, such as a) what the proper pH would be for the optimum proteolytic activity; b) the peptide bond specificity, which is best suited to meet the end product requirements; and c) whether or not the substrate requires debittering. The preferred enzyme for the protein wheat gluten is a neutral endoprotease, specifically Prozyme® 6 (Amano International Enzyme, Troy, Virgina).

The enzyme hydrolysis of the protein occurs at a temperature of from about 25 to about 75°C and at a pH of from about 5.5 to about 8.5, with a neutral enzyme present in the amount of from about 0.1% to about 2.0% by weight of the substrate. Again, these conditions will vary depending on the protein-protease combination. For example, the pH is dependent upon the type of enzyme used. If an acidic enzyme is used, the pH will be in the range of from about 1.5 to about 4.0 and if an alkaline enzyme is used, the pH will be in the range of from about 7.0 to about 12.0. The present pH range is based on the use of a neutral protease.

For the preferred case of wheat gluten and Prozyme 6®, a neutral protease, the enzyme hydrolysis is carried out at a temperature of from about 40 to about 50°C, preferably 45°C, and at a pH of from about 6.5 to about 7.0, with a preferred level from about 0.5% to 1.0% by weight Prozyme 6®. The time during which the enzyme hydrolysis takes place is dependent upon quite a number of factors, specifically, the enzyme concentration used, the pH, the temperature of the reaction, the substrate level and the desired degree of hydrolysis. For the preferred embodiment, a time period of about four (4) hours is suggested.

The substrate level also plays an important role in the present process. The desired level is from about 1.0 to about 30% by weight of the total batch, with the preferred level from about 22 to about 26% by weight. These levels are exceptionally high, and generally cannot be achieved by conventional methods. In order to reach to desired levels, the substrate is added to the enzyme, instead of the conventional method of adding the enzyme to the substrate.

The enzyme hydrolysis is designed to accomplish a major portion of the peptide bond hydrolysis, which is necessary to release the flavor active peptides and amino acids. It does not release the glutamic acid, or monosodium glutamate, from glutamine, nor does it act on the amide bonds of the glutamine which is bound to the peptides. As stated above, quite a range of commercially available endoproteases and exoproteases may be used to achieve the desired result. Specific exoproteases, such as Debitrase® (Imperial Biotechnology Inc., Rosemont, Illinois), which contains leucine amino peptidase, may be used if it is desired to reduce the bitterness from hydrophobic peptides which are present in the hydrolyzed vegetable protein.

It must be pointed out that an endoprotease is absolutely necessary to carry out the initial enzyme hydrolysis. Therefore, if only one protease is used, it must be an endoprotease. If more than one enzyme is used to hydrolyze the vegetable protein the enzymes may be any combination of endoproteases and exoproteases, and they may be used either simultaneously or sequentially.

At this point, the enzymatic process may be stopped at the desired stage by the addition of acid to the aqueous solution. This step is not a necessary one, although it is part of the preferred process, and the hydrolyzed soluble protein can be separated from the insoluble mass without it. However, addition of a food grade organic or inorganic acid to bring the aqueous solution to a pH of from about 2.0 to about 4.0 will stop the enzymatic reaction, thereby providing precise control of the end point, and providing microbiological stability to the hydrolyzate. Addition of a food grade acid at the desired time will also provide for better separation of the hydrolyzed soluble protein from the insoluble mass. The acid may be added once the hydrolyzate has reached the desired degree of solubility and degree of hydrolysis. Specifically, the degree of solubility should, for economic reasons, be at least 60%, with a preferred level of at least 90%. The degree of hydrolysis should be in the range of from about 10 to about 70%, preferably about 20% to about 50%.

The hydrolyzed vegetable protein is then separated from the insoluble mass by any suitable, conventional method, such as filtration or centrifugation or combinations thereof.

Thereafter, the hydrolyzed soluble protein is subjected to mild acid hydrolysis by the addition of a food grade acid and is heated. The acid may be mineral acid alone, or it may be combined with an organic acid. This mild acid hydrolysis is designed to maximize deamidation of free amino acids and peptides and minimize the formation of pyroglutamic acid, through optimization of the level of excess hydrogen ion concentration. Specifically, after titrating the hydrolyzed protein, the excess hydrogen ion concentration is from about 0.5 to about 2.0 molar, preferably 1.0 molar. Examples of food grade acids which can be used in this step include hydrochloric acid, phosphoric acid, sulphuric acid and any combination thereof. In addition, these mineral acids may be partially replaced by various organic acids such as malic acid. The preferred acid for use in the present invention is hydrochloric acid. This deamidation step is carried out at a temperature of from about 75 to about 100°C, preferably about 95°C. The acidified hydrolyzate from the deamidation is then neutralized to a pH of from about 5.0 to about 7.0. Any number of known agents can be used, but the preferred agent is food grade 50% sodium hydroxide.

The resultant neutralized hydrolyzate may then be further processed, if desired, to get it into a more usable form. The hydrolyzate can be subjected to decolorization and deodorization processes. This is conventionally carried out by the use of activated carbon. The decolorized, deodorized hydrolyzate may then be concentrated. This can be performed by any number of methods currently known, such as spray drying, vacuum tray drying or evaporation, for example by a falling thin film evaporator.

During the mild acid deamidation, all the glutamine produced by the initial enzymatic process is converted to monosodium glutamate (MSG). Therefore, the amount of MSG present in the final product is determined by the enzymatic process followed and the substrate used. For example, if wheat gluten is the vegetable protein in use and the enzymatic process goes to total conversion, the level of MSG in the final product can be as high as about 20 to 25% by weight on a dry weight basis.

The following are examples further illustrate the present invention:

EXAMPLE I

Enzyme Hydrolysis

Each sample was subjected to enzyme hydrolysis in a New Brunswick scientific MICROFERM fermenter equipped with a 14 liter vessel and a standard configuration. The general procedure followed for conducting enzyme hydrolysis of wheat gluten was to first charge the reaction vessel with 65 to 90% of the total water to be charged. While the vessel was being brought up to temperature, the pH electrode was standardized and the autotitrator was charged with 4.0M sodium hydroxide. The titrator was set to the target pH and the enzyme solution (10% enzyme w/w in D.I. water) was prepared.

2400 grams of wheat gluten (Manildra Milling Corp., Shawnee Mission, Kansas 66205) were added to 24 grams of Prozyme 6® (Amano Enzymes, U.S. agent: Mitsubishi International Corp., New York, New York 10022) in 7500 grams of water, over a period of 15 to 20 minutes with constant agitation. The enzymatic hydrolysis, maintained at pH 7 and at a temperature of 45°C was allowed to proceed for 4 hours.

After 4 hours, the hydrolyzate was rapidly titrated to pH 2 with 20 Baume (10.0 N) food grade hydrochloric acid (HCl). The acidified hydrolyzate was then pumped through tubing immersed in ice water into collection vessels and immediately refrigerated. The soluble phase was recovered by centrifuging the entire hydrolyzate for 15 minutes in a centrifuge at 16,000xG, and the recovered supernate was concentrated via freeze drying to prepare it for deamidation.

Mild Acid Deamidation

9.63 grams of the freeze dried supernate (0.1% moisture) were weighed into a 50 mL volumetric flask. 10M HCl was added up to the 50 mL mark (for 1.0M HCl excess, 9.04g 10M HCl were used; for 1.5M HCl excess, 11.94g were used; and for 2.0M HCl excess, 14.84g were used). The sample was transferred to a 125 mL Wheaton serum bottle (wheaton catalog number 223748) and incubated in a shaker water bath for 1 hour. The sample was then neutralized with 50% food grade sodium hydroxide (NaOH; J.F. Henry Chemical Co., Inc., Union, New Jersey 07083). The results are set forth in the table below.

## TABLE I

| SAMPLE | HCl | WATER BATH TEMPERATURE (°C) | DH (%) | DEAMID (%) | GLU (g/L) | PG (g/L) |
|---|---|---|---|---|---|---|
| 1 | 1.0M | 95 | 44.8 | 67.7 | 11.5 | 2.5 |
| 2 | 2.0M | 95 | 48.3 | 106.0 | 12.6 | 1.75 |
| 3 | 1.5M | 85 | NA | NA | 10.1 | 3.1 |
| 4 | 1.0M | 75 | 34.6 | 54.9 | 6.0 | 5.3 |
| 5 | 2.0M | 75 | 39.4 | 65.6 | 8.1 | 3.6 |

DH = Degree of hydrolysis; percent of total peptide bonds hydrolyzed.
Deamid = Percent deamidation based on total ammonia released.
GLU = Free glutamic acid (MSG).
PG = Free pyroglutamic acid.
NA = Not Available.

EXAMPLE II

Enzyme Hydrolysis

Enzyme hydrolysis was carried out following the procedure set forth in Example I.

5

## Mild Acid Deamidation

The basis for this example, was to scale up the deamidation process of Example I to a 1 liter reaction. 686.7 grams of water and 183.3 grams of 10M HCl (1.0M excess) were charged into the reactor and heated to 95°C. 200 grams of hydrolyzate (20% w/v) were then charged into the reactor as dry powder through a side port, over a period of approximately 5 minutes. A condenser was placed in the port and the reaction proceeded for a period of 1 hour at a constant temperature of 95°C. The heat source was then removed from the reaction vessel, and the vessel was rapidly cooled in an ice bath to below 50°C in order to stop the reaction. 160 grams of 50% NaOH were added to the vessel to bring the sample to pH 7. This neutralization was carried out in an ice bath below 25°C to maintain the MSG level. 1100 grams of the neutralized deamidate were then treated with 11 grams of Darco KB® activated carbon (American Norit company, Inc., Jacksonville, Florida) for 2 hours at room temperature. The deamidate was then filtered on a Buchner funnel over Whatman 42 ashless paper. The filtrate was concentrated in a vacuum tray dryer at 50°C and 25 mmHg for 2 hours. The resultant product, which was at a concentration of approximately 70% solids, was diluted with water to produce a homogenous solution of approximately 40% solids for analysis. The MCP level was then measured by standard GC procedure which is sensitive to 2ppm with the result indicating no detectable levels of MCP. MSG was present in the end product at a level of 5% on a dry basis.

## EXAMPLE III

### Enzyme Hydrolysis

Enzyme hydrolysis was carried out following the procedure set forth in Example I.

### Mild Acid Deamidation

The procedure set forth in Example II was followed to see the effect of using 2.0M excess HCl on the deamidation and MCP formation. 200 grams (dry weight) of the hydrolyzate were charged into a reactor containing 570 grams water and 300 grams of 10M HCl (2.0M excess). After 1 hour at 95°C, the deamidate was cooled and neutralized with 232 grams of 50% NaOH. 608 grams of the neutralized deamidate were treated with 6.1 grams Darco S-51® activated carbon for 2 hours at room temperature. The deamidate was then filtered and concentrated. A homogeneous solution of approximately 45% solids was used for analysis by GC, with the result being no detectable level of MCP.

## EXAMPLE IV

### Enzyme Hydrolysis

Enzyme hydrolysis was carried out on the sample following the procedure set forth in Example I.

### Mild Acid Deamidation

The same deamidation procedure was followed, but the enzyme hydrolyzate concentration was increased to see the effect on deamidation and MCP formation. 400 grams of the hydrolyzate (40% w/v) were added to a solution of 250.6 grams of 10M HCl (1.0M excess) in 490.0 grams of water (substrate density = 1.14). After 1 hour at 95°C, the deamidate was cooled and neutralized with 220 grams of 50% NaOH. 647 grams of the neutralized deamidate were treated with 6.5 grams of Darco S-51® activated carbon for 2 hours at room temperature. After filtering and concentrating, a solution of approximately 45% solids was analyzed by a GC, with no detectable level of MCP resulting.

## EXAMPLE V

### Enzyme Hydrolysis

The same procedure set forth in Example I was followed, except that a combination of neutral proteases was used. 24 grams of Prozyme 6® and 33.7 grams (0.5% enzyme/substrate dry basis) of Neutrase liquid (as received) (NOVO Industries, Danbury, Connecticut) were initially charged to the reaction vessel with water.

Mild Acid Deamidation

Following the procedure set forth in Example II, 200 grams of the hydrolyzate were added to a solution of 183.3 grams 10M HCl (1.0M excess) and 686.7 grams water. After 1 hour at 95°C, the cooled deamidate was neutralized with 156 grams 50% NaOH. 400 grams of deamidate were treated with 4 grams of Darco KB® activated carbon for 2 hours at room temperature. After filtering and condensing, a solution of approximately 40% solids was subjected to GC analysis with no detectable level of MCP.

EXAMPLE VI

Enzyme Hydrolysis

The procedure for enzyme hydrolysis set forth in Example I was followed, except that a combination of enzymes were used, and the enzymes were added consecutively. This procedure was followed in order to see the effect of an exopeptidase on releasing MSG. 24 grams of Prozyme 6® were initially charged to the reactor with the water and following the above procedure, 2400 grams of wheat gluten were later charged to the vessel. After 30 minutes, 13.0 grams of Debitrase® 4500.10 (Imperial Biotechnology Inc., Rosemont, Illinois) were added to the reactor and the reaction was allowed to run for a period of 4 hours after the addition of the Debiterase, or a total of 4.5 hours.

Mild Acid Deamidation

Following the procedure of Example II, 200 grams of hydrolyzate were added to a solution of 183.3 grams 10M HCl (1.0M excess) and 686.7 grams water (substrate density = 1.07). After 1 hour at 95°C, the cooled deamidate was neutralized with 159 grams of 50% NaOH. 618 grams of the neutral deamidate were treated with 6.2 grams Darco S-51® carbon for 2 hours at room temperature. An approximately 40% solid solution was analyzed by a GC, with no detectable level of MCP. The level of MSG present in the final product was at a level of 10% by weight on a dry weight basis.

EXAMPLE VII

Enzyme Hydrolysis

The general procedure for enzyme hydrolysis set forth in Example I was followed except that a different enzyme was used resulting in the necessity to alter the time and temperature at which the enzymatic hydrolysis took place. 36 grams of Debitrase® 4060.50 (1.5% enzyme/substrate dry basis) were used as the enzyme in place of Prozyme 6®, and the hydrolysis was carried out at 38°C for a period of 6 hours.

Mild Acid Deamidation

200 grams of hydrolyzate were added to a solution of 183.3 grams 10M HCl (1.0M excess) and 686.7 grams water. After 1 hour at 95°C, 153g of 50% NaOH were added to neutralize the cooled deamidate. 600 grams of the neutralized deamidate were treated with 6.0 grams of Darco S-51® activated carbon for 2 hours at room temperature. A 40% solids sample was analyzed with the resultant MCP level being non-detectable.

**Claims**

1.  A process for the production of hydrolyzed vegetable proteins containing no detectable level of monochloropropanol, which means that there is no detectable level as measured by gas chromatography (GC) with a sensitivity to levels as low as 2 ppm, which comprises:
    (a) hydrolyzing a vegetable protein by adding it to an aqueous solution of at least one protease, which is at least an endoprotease and optionally another protease;
    (b) separating the hydrolyzed soluble protein from the insoluble mass;
    (c) adding acid to the hydrolyzed soluble protein and heating the mixture to substantially deamidate the hydrolyzate, whereby the mild acid hydrolysis is carried out for a time period short enough to yield no detectable level of monochloropropanol, which means that there is no detectable level as measured by gas chromatography with a sensitivity to levels as low as 2 ppm; and

(d) neutralizing the deamidated hydrolyzate.

2. The process of claim 1 which further comprises addition of an acid to the aqueous solution in step (a) to stop the enzymatic reaction.

3. The process of claim 1 which further comprises deodorization and decoloration of the hydrolyzate from step (d).

4. The process of claim 3 which further comprises concentrating the deodorized and decolorized hydrolyzate.

5. The process of claim 1 wherein the protease in step (a) is an endoprotease.

6. The process of claim 5 wherein the endoprotease is acidic, neutral or alkaline.

7. The process of claim 1 wherein the vegetable protein in step (a) is chosen from the group consisting of oil seed proteins, plasma proteins, leaf proteins or combinations thereof.

8. The process of claim 7 wherein the vegetable protein is wheat gluten.

9. The process of claim 1 wherein the hydrolysis in step (a) takes place at a temperature of from 25 to 75°C and a pH of from 5.5 to 8.5.

10. The process of claim 9 wherein the hydrolysis in step (a) takes place at a temperature of from 40 to 50°C and a pH of from 6.5 to 7.0.

11. The process of claim 1 wherein the separation in step (b) is by filtration, centrifugation or combinations thereof.

12. The process of claim 1 wherein the added acid in step (c), after titrating the hydrolyzed protein, produces an excess hydrogen ion concentration of from 0.5 to 2.0 molar.

13. The process of claim 12 wherein the added acid in step (c) produces an excess hydrogen ion concentration of 1.0 molar.

14. The process of claim 1 wherein the reaction in step (c) takes place at a temperature of from about 75 to 100°C.

15. The process of claim 14 wherein the reaction in step (c) takes place at a temperature of 95°C.

16. The process of claim 1 wherein the neutralization of step (d) takes place at a pH of from 5.0 to 7.0.

17. The process of claim 1 wherein the hydrolysis in step (a) is effected by the sequential addition of at least two proteases.

18. The process of claim 1 wherein the hydrolysis in step (a) is effected by the simultaneous addition of at least two proteases.

19. A process for the production of hydrolyzed vegetable proteins containing no detectable level of monochloropropanol according to claim 1, which comprises in addition to steps (a) to (d) of claim 1 the following steps:
(e) adding an acid to the aqueous solution of step (a) to stop the enzymatic reaction;
(f) decolorizing and deodorizing the hydrolyzate from step (d); and
(g) concentrating the hydrolyzate from step (f).

20. The process of claim 19 wherein the vegetable protein in step (a) is chosen from the group consisting of oil seed proteins, plasma proteins, leaf proteins or combinations thereof.

21. The process of claim 20 wherein the vegetable protein in step (a) is wheat gluten.

22. The process of claim 19 wherein the deodorization and decolorization of step (f) is carried out by the use of activated carbon.

23. The process of claim 19 wherein the acid in step (e) is a food grade mineral acid.

24. The process of claim 19 wherein the acid in step (e) is a combination of food grade mineral and organic acids.

25. The process of claim 19 wherein the acid in step (c) is a food grade mineral acid.

26. The process of claim 19 wherein the acid in step (c ) is a combination of food grade mineral and organic acids.

27. The process of claim 24 wherein the food grade mineral or organic acid is chosen from the group consisting of hydrochloric acid, phosphoric acid, sulphuric acid, citric acid, malic acid and combinations thereof.

28. The product of the process of claim 1 or of claim 19.

29. A hydrolyzed vegetable protein which has no detectable level of monochloropropanol, which means that there is no detectable level as measured by gas chromatography (GC) with a sensitivity to levels as low as 2 ppm, which is clean and bland in flavor and which exhibits substantial flavor enhancement characteristics.

30. The hydrolyzed vegetable protein of claim 29 which comprises substantial amounts of monosodium glutamate, up to 25% by weight on a dry weight basis.


**Patentansprüche**

1. Verfahren zur Herstellung von hydrolysierten pflanzlichen Proteinen, welche keine nachweisbare Menge an Monochlorpropanol enthalten, was bedeutet, dass keine nachweisbare Menge, gemessen durch Gaschromatographie (GC) mit einer Empfindlichkeit auf Mengen bis hinunter auf 2 ppm, vorhanden ist, welches umfasst :
   (a) das Hydrolysieren eines pflanzlichen Proteins, indem man es zu einer wässerigen Lösung von mindestens einer Protease zusetzt, welche mindestens eine Endoprotease und gegebenenfalls eine weitere Protease ist;
   (b) Trennung des hydrolysierten löslichen Proteins von der unlöslichen Masse;
   (c) Zusatz von Säure zu dem hydrolysierten löslichen Protein und Erhitzen des Gemisches um das Hydrolysat im wesentlichen zu desamidieren, wobei die milde Säurehydrolyse während einer Zeit durchgeführt wird, welche kurz genug. ist, um keine nachweisebare Menge an Monochlorpropanol. zu ergeben, was bedeutet, dass keine nachweisbare Menge, wie durch Gaschromatographie mit einer Empfindlichkeit auf Mengen bis hinter auf 2 ppm gemessen, zugegen ist, und
   (d) Neutralisieren des desamidierten Hydrolysates.

2. Verfahren nach Anspruch 1, welches ferner den Zusatz einer Säure zu der wässerigen Lösung in Stufe (a) umfasst, um die enzymatische Reaktion zu unterbrechen.

3. Verfahren nach Anspruch 1, welches ferner die Desodorierung und Entfärbung des Hydrolysates aus Stufe (d) umfasst.

4. Verfahren nach Anspruch 3, welches ferner das Konzentrieren des desodorierten und entfärbten Hydrolysates umfasst.

5. Verfahren nach Anspruch 1, in welchem die Protease in Stufe (a) eine Endoprotease ist.

6. Verfahren nach Anspruch 5, in welchem die Endoprotease sauer, neutral oder alkalisch ist.

7. Verfahren nach Anspruch 1, in welchem das pflanzliche Protein in Stufe (a) ausgewählt ist aus der Gruppe bestehend aus Oelsamenproteinen, Plasmaproteinen, Blattproteinen oder Kombinationen davon.

8. Verfahren nach Anspruch 7, in welchem das pflanzliche Protein Weizengluten ist.

9. Verfahren nach Anspruch 1, in welchem die Hydrolyse in Stufe (a) bei einer Temperatur von 25 bis 75°C und einem pH von 5,5 bis 8,5 stattfindet.

10. Verfahren nach Anspruch 9, in welchem die Hydrolyse in Stufe (a) bei einer Temperatur von 40 bis 50°C und einem pH von 6,5 bis 7,0 stattfindet.

11. Verfahren nach Anspruch 1, in welchem die Trennung in Stufe (b) durch Filtration, Zentrifugieren oder Kombinationen davon erfolgt.

12. Verfahren nach Anspruch 1, in welchem die zugesetzte Säure in Stufe (c) nach Titration des hydrolysierten Proteins einem Wasserstoffionenkonzentrationsüberschuss von 0,5 bis 2,0 molar ergibt.

13. Verfahren nach Anspruch 12, in welchem die zugesetzte Säure in Stufe (c) einen Wasserstoffionenkonzentrationsüberschuss von 1,0 molar ergibt.

14. Verfahren nach Anspruch 1, in welchem die Reaktion der Stufe (c) bei einer Temperatur von etwa 75 bis 100°C erfolgt.

15. Verfahren nach Anspruch 14, in welchem die Reaktion in Stufe (c) bei einer Temperatur von 95°C erfolgt.

16. Verfahren nach Anspruch 1, in welchem die Neutralisation der Stufe (d) bei einem pH von 5,0 bis 7,0 erfolgt.

17. Verfahren nach Anspruch 1, in welchem die Hydrolyse in Stufe (a) durch aufeinanderfolgenden Zusatz von mindestens zwei Proteasen bewirkt wird.

18. Verfahren nach Anspruch 1, in welchem die Hydrolyse in Stufe (a) durch gleichzeitigen Zusatz von mindestens zwei Proteasen bewirkt wird.

19. Verfahren für die Herstellung von hydrolysierten pflanzlichen. Proteinen, welche keine nachweisbare Menge an Monochlorpropanol enthalten, gemäss Anspruch 1, welches zusätzlich zu den Stufen (a) bis (d) von Anspruch 1 die folgenden Stufen umfasst :
   (e) Zusatz einer Säure zu der wässerigen Lösung in Stufe (a) um die enzymatische Reaktion zu unterbrechen;
   (f) Entfärbung und Desodorierung des Hydrolysates aus Stufe (d); und
   (g) Konzentrieren des Hydrolysates aus Stufe (f) .

20. Verfahren nach Anspruch 19, in welchem das pflanzliche Protein in Stufe (a) ausgewählt ist aus der Gruppe bestehend aus Oelsamenproteinen, Plasmaproteinen, Blattproteinen oder Kombinationen davon.

21. Verfahren nach Anspruch 20, in welchem das pflanzliche Protein in Stufe (a) Weizengluten ist.

22. Verfahren nach Anspruch 19, in welchem die Desodorierung und Entfärbung der Stufe (f) unter Verwendung von Aktivkohle durchgeführt wird.

23. Verfahren nach Anspruch 19, in welchem die Säure in Stufe (e) eine Mineralsäure von Nahrungsmittelqualität ist.

24. Verfahren nach Anspruch 19, in welchem die Säure in Stufe (e) eine Kombination von Mineralsäuren und organischen Säuren von Nahrungsmittelqualität ist.

25. Verfahren nach Anspruch 19, in welchem die Säure in Stufe (c) eine Mineralsäure von Nahrungsmittelqualität ist.

26. Verfahren nach Anspruch 19, in welchem die Säure in Stufe (c) eine Kombination von Mineralsäuren und organischen Säuren von Nahrungsmittelqualität ist.

27. Verfahren nach Anspruch 24, in welchem die Mineral- oder organische Säure von Nahrungsmittelqualität ausgewählt ist aus der Gruppe bestehend aus Salzsäure, Phosphorsäure, Schwefelsäure, Zitronensäure, Apfelsäure und Kombinationen davon.

28. Produkt des Verfahrens nach Anspruch 1 oder Anspruch 19.

29. Hydrolysiertes pflanzliches Protein, welches keine nachweisbare Menge an Monochlorpropanol enthält, was bedeutet, dass keine nachweisbare Menge, gemessen durch Gaschromatographie (GC) mit einer Empfindlichkeit auf Mengen bis hinab auf 2 ppm, zugegen ist, welches sauber und angenehm im Geschmack ist und welches wesentliche geschmacksverbessernde Eigenschaften aufweist.

30. Hydrolysiertes pflanzliches Protein nach Anspruch 29, welches wesentliche Mengen an Mononatriumglutamat, bis zu 25 Gewichtsprozent, bezogen auf das Trockengewicht, enthält.

**Revendications**

1. Procédé de production de protéines végétales hydrolysées ne contenant aucune teneur détectable de monochloropropanol, expression signifiant qu'il n'existe aucune teneur détectable telle qu'elle est mesurée par chromatographie en phase gazeuse (CG) avec une sensibilité à des teneurs pouvant s'abaisser à 2 ppm, qui comprend :
   (a) l'hydrolyse d'une protéine végétale par addition de cette protéine à une solution aqueuse d'au moins une protéase, qui consiste en au moins une endoprotéase et, facultativement, une autre protéase ;
   (b) la séparation de la protéine soluble hydrolysée de la masse insoluble ;
   (c) l'addition d'un acide à la protéine soluble hydrolysée et le chauffage du mélange pour désamider fortement l'hydrolysat, l'hydrolyse acide douce étant ainsi effectuée pendant un temps suffisamment bref pour ne donner aucune teneur détectable de monochloropropanol, ce qui signifie qu'il n'existe aucune teneur détectable telle qu'elle est mesurée par chromatographie en phase gazeuse avec une sensibilité à des teneurs pouvant s'abaisser à 2 ppm ; et
   (d) la neutralisation de l'hydrolysat désamidé.

2. Procédé suivant la revendication 1, qui comprend en outre l'addition d'un acide à la solution aqueuse dans l'étape (a) pour arrêter la réaction enzymatique.

3. Procédé suivant la revendication 1, qui comprend en outre une désodorisation et une décoloration de l'hydrolysat de l'étape (d).

4. Procédé suivant la revendication 3, qui comprend en outre la concentration de l'hydrolysat désodorisé et décoloré.

5. Procédé suivant la revendication 1, dans lequel la protéase de l'étape (a) est une endoprotéase.

6. Procédé suivant la revendication 5, dans lequel l'endoprotéase est acide, neutre ou alcaline.

7. Procédé suivant la revendication 1, dans lequel la protéine végétale dans l'étape (a) est choisie dans le groupe consistant en protéines de graines oléagineuses, protéines plasmatiques, protéines de feuilles ou leurs associations.

8. Procédé suivant la revendication 7, dans lequel la protéine végétale est le gluten de blé.

9. Procédé suivant la revendication 1, dans lequel l'hydrolyse dans l'étape (a) s'effectue à une température de 25 à 75°C et à un pH de 5,5 à 8,5.

10. Procédé suivant la revendication 9, dans lequel l'hydrolyse dans l'étape (a) s'effectue à une température de 40 à 50°C et à un pH de 6,5 à 7,0.

11. Procédé suivant la revendication 1, dans lequel la séparation dans l'étape (b) est effectuée par filtration, centrifugation ou leurs associations.

12. Procédé suivant la revendication 1, dans lequel l'acide ajouté dans l'étape (c), après titrage de la protéine hydrolysée, produit une concentration d'ions hydrogène en excès de 0,5 à 2,0 molaire.

13. Procédé suivant la revendication 12, dans lequel l'acide ajouté dans l'étape (c) produit une concentration d'ions hydrogène en excès de 1,0 molaire.

14. Procédé suivant la revendication 1, dans lequel la réaction dans l'étape (c) s'effectue à une température d'environ 75 à 100°C.

15. Procédé suivant la revendication 14, dans lequel la réaction dans l'étape (c) s'effectue à une température de 95°C.

16. Procédé suivant la revendication 1, dans lequel la neutralisation de l'étape (d) s'effectue à un PH de 5,0 à 7,0.

17. Procédé suivant la revendication 1, dans lequel l'hydrolyse dans l'étape (a) est effectuée par addition successive d'au moins deux protéases.

18. Procédé suivant la revendication 1, dans lequel l'hydrolyse dans l'étape (a) est effectuée par addition simultanée d'au moins deux protéases.

19. Procédé de production de protéines végétales hydrolysées ne contenant aucune teneur détectable de monochloropropanol suivant la revendication 1, qui comprend, en plus des étapes (a) à (d) de la revendication 1, les étapes suivantes :
    (e) l'addition d'un acide à la solution aqueuse de l'étape (a) pour arrêter la réaction enzymatique ;
    (f) la décoloration et la désodorisation de l'hydrolysat de l'étape (d) ; et
    (g) la concentration de l'hydrolysat de l'étape (f).

20. Procédé suivant la revendication 19, dans lequel la protéine végétale dans l'étape (a) est choisie dans le groupe consistant en protéines de graines oléagineuses, protéines plasmatiques, protéines de feuilles ou leurs associations.

21. Procédé suivant la revendication 20, dans lequel la protéine végétale dans l'étape (a) est le gluten de blé.

22. Procédé suivant la revendication 19, dans lequel la désodorisation et la décoloration de l'étape (f) sont conduites au moyen de charbon activé.

23. Procédé suivant la revendication 19, dans lequel l'acide dans l'étape (e) est un acide minéral à usage alimentaire.

24. Procédé suivant la revendication 19, dans lequel l'acide dans l'étape (e) est une association d'acides minéraux et organiques à usage alimentaire.

25. Procédé suivant la revendication 19, dans lequel l'acide dans l'étape (c) est un acide minéral à usage alimentaire.

26. Procédé suivant la revendication 19, dans lequel l'acide dans l'étape (c) est une association d'acides minéraux et organiques à usage alimentaire.

27. Procédé suivant la revendication 24, dans lequel l'acide minéral ou organique à usage alimentaire est choisi dans le groupe consistant en acide chlorhydrique, acide phosphorique, acide sulfurique, acide citrique, acide malique et leurs associations.

28. Produit obtenu par mise en oeuvre du procédé suivant la revendication 1 ou la revendication 19.

29. Protéine végétale hydrolysée qui ne possède aucune teneur détectable de monochloropropanol, ce qui signifie qu'il n'existe aucune teneur détectable telle qu'elle est mesurée par chromatographie en phase gazeuse (CG) avec une sensibilité à des teneurs pouvant s'abaisser à 2 ppm, qui est pure et de saveur douce et qui présente des caractéristiques notables de renforcement de saveur.

30. Protéine végétale hydrolysée suivant la revendication 29, qui comprend des quantités importantes de glutamate monosodique, allant jusqu'à 25 % en poids sur base pondérale sèche.